# EUROPÄISCHE PATENTSCHRIFT

(11) **EP 1 646 344 B1**
(45) Veröffentlichungstag und Bekanntmachung des Hinweises auf die Patenterteilung: **12.02.2014**
(21) Anmeldenummer: 04741209.3
(22) Anmeldetag: 21.07.2004
(51) Int. Cl.: A61F 13/15, A61L 15/16, A61F 13/20

(54) **DENTALES EINLEGEMITTEL MIT NICHTZYLINDRISCHER FORM**
DENTAL INLAY MEANS HAVING A NON-CYLINDRICAL SHAPE
MOYEN ABSORBANT A USAGE DENTAIRE DE FORME NON CYLINDRIQUE

(30) Priorität: 22.07.2003 DE 10333455
(43) Veröffentlichungstag der Anmeldung: 19.04.2006
(73) Patentinhaber: Coltène/Whaledent GmbH + Co. KG, 89129 Langenau (DE)
(72) Erfinder: MÜLLER, Barbara, 89129 Langenau (DE); MANNSCHEDEL, Werner, 89129 Langenau (DE)
(74) Vertreter: Forstmeyer, Dietmar
(86) Internationale Anmeldenummer: PCT/EP2004/008178
(87) Internationale Veröffentlichungsnummer: WO 2005/009310

(56) Entgegenhaltungen:
- EP-A- 1 125 570
- WO-A-96/20683
- US-A- 3 347 237
- US-A- 3 607 520
- US-A- 3 674 030
- US-A- 4 372 314
- US-A- 4 533 357
- US-A- 4 705 514
- DATABASE WPI Section Ch, Week 200407 Derwent Publications Ltd., London, GB; Class D22, AN 2004-065127 XP002304778 & JP 2003 180741 A (KAO CORP) 2. Juli 2003 (2003-07-02) & PATENT ABSTRACTS OF JAPAN Bd. 2003, Nr. 11, 5. November 2003 (2003-11-05) & JP 2003 180741 A (KAO CORP), 2. Juli 2003 (2003-07-02)

## Beschreibung

Die vorliegende Erfindung liegt allgemein auf dem Gebiet der Hilfsmittel für die Zahnheilkunde und betrifft ein dentales Einlegemittel mit nichtzylindrischer Form zur Anwendung in der Mundhöhle.

Bei zahnmedizinischen Eingriffen, wie etwa einer Wurzelbehandlung oder einer Kavitätenbehandlung, ist es für den Erfolg einer Behandlung von wesentlicher Bedeutung, den Behandlungsbereich im Mund des Patienten sauber und trocken zu halten. Eine Vielzahl von im Dentalbereich zu Behandlungszwecken eingesetzte Materialien können durch einen unerwünschten Kontakt mit wässrigen Substanzen, wie etwa Speichelflüssigkeit oder Blut, in nachteiliger Weise in ihren Eigenschaften wesentlich beeinträchtigt werden. Ferner kann es durch einen derartigen Kontakt zu Infektionen kommen.

Eine oft angewandte Möglichkeit zur Lösung dieses Problems besteht beispielsweise darin, einen Kofferdam-Spanngummi anzulegen. Hierbei wird die Umgebung des Behandlungsbereichs mithilfe eines mit einem Loch versehenen Spanngummis abgedeckt. Bei manchen Patienten kann es allerdings bei Einsatz eines Kofferdam-Spanngummis zu allergischen Reaktionen kommen.

Schneller anzuwenden und für den Patienten weitaus bequemer ist die bekannte Verwendung von dentalen Einlegemitteln, wie beispielsweise aus Baumwolle gefertigten Watterollen. Die Herstellung derartiger im Stand der Technik bekannter Watterollen ist einfach. Gewöhnlich wird hierzu z. B. eine Lage Baumwoll-Vlies spiralig gerollt und dann mithilfe eines geeigneten Klebstoffs auf der Außenseite verklebt. Anschließend werden die Watterollen in der gewünschten Länge abgelängt.

Neben deren Eigenschaft Flüssigkeiten aufzunehmen, sind Watterollen geeignet, Mundschleimhaut bzw. Zunge und den zu behandelnden Zahnbereich voneinander zu trennen, wobei dieser Abstandshalterfunktion eine besonders wichtige Rolle zukommt, da Flüssigkeiten im Mundraum während der Behandlung heutzutage regelmäßig durch gesonderte Saugvorrichtungen abgesaugt werden.

Nun hat sich aber gezeigt, dass herkömmliche Watterollen, welche beispielweise zwischen Backenschleimhaut und Zähne geklemmt werden, leicht verrutschen, insbesondere wegrollen, können, wodurch die Backenschleimhaut in unerwünschter Weise Kontakt mit den Stellen erhalten kann, von welchen sie aus oben genannten Gründen getrennt werden sollte. Darüber hinaus ist die Abstandshalterfunktion aufgrund des geringen Durchmessers der Watterollen oftmals unzureichend, zumal sich die Watterollen bei Durchfeuchtung und unter Druck zu einem Oval verformen.

Weiterhin hat sich als nachteilig erwiesen, dass Watterollen, bei bestimmten Anwendungen, wie etwa beim herkömmlichen Bleichen der Zähne bei Zahnverfärbungen, nur bedingt taugen, da eine vollständige Abdeckung von nicht mit Bleichmittel zu kontaktierenden Stellen nicht gewährleistet ist.

Ferner haben Watterollen den Nachteil einer nur bedingten Stapelfähigkeit bei der Lagerung.

Demgegenüber besteht die Aufgabe der vorliegenden Erfindung darin, ein dentales Einlegemittel zur Verfügung zu stellen, welches zuverlässig im Mundraum angebracht werden kann, ohne dass ein Verrutschen zu befürchten ist, und durch welches gegenüber den herkömmlichen Watterollen eine verbesserte Abstandshalterfunktion erzielt werden kann. Ferner soll durch ein derartiges dentales Einlegemittel vorzugsweise ein nahezu vollständiges Abdecken bestimmter Bereiche der Mundhöhle bzw. der Zähne gewährleistet werden können, um den Einsatz eines derartigen dentalen Einlegemittels bei bestimmten zahnärztlichen Tätigkeiten, wie etwa dem Bleichen von Zahnverfärbungen, zu ermöglichen. Nicht zuletzt soll die Lagerung, insbesondere Stapelfähigkeit, eines solchen dentalen Einlegemittels gegenüber den herkömmlichen Watterollen verbessert sein.

Diese Aufgabe wird erfindungsgemäß durch den unabhängigen Anspruch gelöst. Vorteilhafte Ausgestaltungen der Erfindung sind in den Unteransprüchen angegeben.

Erfindungsgemäß ist ein dentales Einlegemittel zur Anwendung in der Mundhöhle gezeigt, welches dadurch gekennzeichnet ist, dass es eine, insbesondere senkrecht zur Längsrichtung geschnittene, Querschnittsfläche mit einem allgemein nichtkreisförmigen Umfang aufweist. Das erfindungsgemäße dentale Einlegemittel unterscheidet sich somit in der dreidimensionalen Form von der herkömmlichen zylindrischen Watterolle, und kann allgemein jede beliebige nichtzylindrische dreidimensionale Form aufweisen.

Bei einer besonders vorteilhaften Ausführungsform der vorliegenden Erfindung weist das dentale Einlegemittel eine insbesondere senkrecht zur Längsrichtung geschnittene Querschnittsfläche auf, welche einen Umfang in Gestalt eines ersten Teilkreises mit einem ersten Krümmungsradius aufweist, dessen Endpunkte durch wenigstens eine gekrümmte Linie verbunden sind. Die dreidimensionale Form des dentalen Einlegemittels dieser Ausführungsform weist somit, entsprechend dem im Querschnitt gesehenen ersten Teilkreis, einen längs der Zylinderachse geschnittenen Teilzylinder auf, welcher, entsprechend der im Querschnitt gesehenen wenigstens einen gekrümmten Linie in einen beliebig geformten, gekrümmten Abschnitt übergeht.

Vorteilhaft liegt hierbei, im Querschnitt zur Längsrichtung betrachtet, die die Endpunkte des ersten Teilkreises verbindende gekrümmte Linie in Gestalt eines zweiten Teilkreises vor, wobei der Krümmungsradius des ersten Teilkreises kleiner ist, als der Krümmungsradius des zweiten Teilkreises. Hieraus folgt, dass sich die dreidimensionale Form dieser Ausführungsform des dentalen Einlegemittels aus zwei längs der Zylinderachse geschnittenen Teilzylindern mit verschiedenen Zylinderradien zusammensetzt. Hierbei hat es sich als besonders vorteilhaft erwiesen, wenn der Krümmungsradius des zweiten Teilkreises wesentlich größer ist als der Krümmungsradius des ersten Teilkreises, d. h. wenn der zu dem zweiten Teilkreis gehörende Teilzylinder wesentlich weniger gekrümmt ist als der zu dem ersten Teilkreis gehörende Teilzylinder.

Bei einer weiteren besonders vorteilhaften Ausführungsform des erfindungsgemäßen dentalen Einlegemittels weist die senkrecht zur Längsrichtung geschnittene Querschnittsfläche einen Umfang in Gestalt eines ersten Teilkreises, dessen Endpunkte durch wenigstens eine wenigstens annähernd gerade Linie verbunden sind, auf. Hieraus folgt, dass sich die dreidimensionale Form des dentalen Einlegemittels aus einem längs der Zylinderachse geschnittenen Teilzylinder, welcher in eine oder mehrere annähernd ebene Flächen übergeht, zusammensetzt.

Bei den oben genannten erfindungsgemäßen Ausführungsformen ist es besonders vorteilhaft, wenn die die Endpunkte des ersten Teilkreises verbindende, gekrümmte oder wenigstens annähernd gerade Linie des geometrischen Umfangs der Querschnittsfläche zumindest in der mathematischen Idealisierung nicht stetig differenzierbar in den ersten Teilkreis übergeht. Dies bedeutet, dass die dreidimensionale Form des dentalen Einlegemittels, dort wo der längs der Zylinderachse geschnittene Teilzylinder in den gekrümmten Abschnitt bzw. in die wenigstens eine ebene Fläche übergeht, eine Kante aufweist.

Bei einer weiteren bevorzugten Ausführungsform des erfindungsgemäßen dentalen Einlegemittels weist die senkrecht zur Längsrichtung geschnittene Querschnittsfläche einen geometrischen Umfang in Gestalt eines geschlossenen Polygonzugs auf. Der geometrische Umfang der Querschnittsfläche kann insbesondere in Form eines Ovals, einer Bienenwabenstruktur, eines Rechtecks oder Dreiecks vorliegen.

Ferner ist es vorteilhaft, wenn das erfindungsgemäße dentale Einlegemittel eine gekrümmte dreidimensionale Form aufweist. Gleichermaßen kann es vorteilhaft sein, wenn das erfindungsgemäße dentale Einlegemittel mit einer in Längsrichtung sich verjüngenden dreidimensionalen Form versehen ist.

Bei einer weiteren bevorzugten Ausführungsform des dentalen Einlegemittels weist dieses in einer ersten Schnittebene, welche beispielsweise einer Draufsicht auf das dentale Einlegemittel entspricht, eine im Wesentlichen V-förmige Gestalt auf. Mithilfe einer solchen Gestaltung des dentalen Einlegemittels kann in besonders vorteilhafter Weise nach dessen Platzierung im Mund ein Verschieben der Zunge erreicht werden, so dass Platz für den behandelnden Zahnarzt geschaffen wird. In dieser Hinsicht ist es zudem von Vorteil, wenn ein solches dentales Einlegemittel in einer zur ersten Schnittebene senkrechten zweiten Schnittebene im Wesentlichen keilförmig ausgebildet ist.

Ein derartiges dentales Einlegemittel kann aus einer Lage und insbesondere aus einem mehrlagigen Verbundmaterial geformt sein, wobei es vorteilhaft ist, wenn die Decklagen des Verbundmaterials ein saugfähiges Material enthalten oder daraus bestehen. Zu diesem Zweck kann das Verbundmaterial aus einer Kernlage und mindestens einer, vorzugsweise zwei die Kernlage umgebenden Decklagen zusammengesetzt sein, wobei das Kernmaterial aus wenigstens einem Material ausgewählt aus Silikon, Naturfasern, Synthetikfasern und Kunststoffen, insbesondere Polyethylen, Polyamid oder Polypropylen und gegebenenfalls üblichen Zusatzstoffen, bestehen kann oder diese umfasst, während die Decklagen unabhängig voneinander aus wenigstens einem Material ausgewählt aus saugfähigen natürlichen Fasern und saugfähigen synthetischen Fasern, wie Watte, insbesondere Baumwollwatte und gegebenenfalls üblichen Zusatzstoffen bestehen können oder diese umfassen. Bevorzugt wird ein dentales Einlegemittel, das eine Kernlage und zwei Decklagen aufweist. Die Decklagen können in an sich üblicher Weise, z.B. durch Verkleben auf der Kernlage angebracht werden. Weist das erfindungsgemäße dentale Einlegemittel nur eine Lage auf, so kann diese Lage eines oder eine beliebige Kombination von zwei oder mehr der vorstehend genannten Materialien umfassen oder aus ihnen bestehen.

Bei einer weiteren besonders vorteilhaften Ausführungsform des erfindungsgemäßen dentalen Einlegemittels weist dieses wenigstens eine Fläche auf, welche bestimmten anatomischen Gegebenheiten in der Mundhöhle angepasst ist. So kann wenigstens eine Fläche des dentalen Einlegemittels der Kontur eines Zahns oder mehrerer Zähne angepasst sein. Ebenso kann die Fläche der Zunge und/oder der Innenwand der Mundhöhle, z.B. dem Gaumen angepasst sein. Insbesondere in Verbindung mit einer gekrümmten bzw. in Längsrichtung sich verjüngenden dreidimensionalen Form des dentalen Einlegemittels, lässt sich auf diese Weise ein anatomischen Gegebenheiten "maßgeschneidertes" dentales Einlegemittel fertigen.

Bei allen oben genannten bevorzugten Ausführungsformen des erfindungsgemäßen dentalen Einlegemittels, welche gegebenenfalls alleine oder in Kombination vorliegen können, kann die Gefahr eines Verrutschens, insbesondere Wegrollens, des in der Mundhöhle angebrachten, beispielsweise zwischen Mundschleimhaut und Zähne eingeklemmten, nichtzylindrischen dentalen Einlegemittels verringert werden. Dies gilt in besonderem Maße für jene Ausführungsformen, welche in ihrer dreidimensionalen Form mit Kanten versehen sind. Weiterhin kann durch das erfindungsgemäße dentale Einlegemittel die Abstandshalterfunktion beträchtlich verbessert werden, da das Verhältnis des seitlichen End-zu-End-Abstands des dentalen Einlegemittels in Bezug auf dessen Volumen gegenüber der herkömmlichen zylindrischen Watterolle vergrößert werden kann. Dies gilt in besonderem Maße für jene Ausführungsformen, welche eine dreidimensionale Form in Gestalt eines Teilzylinders, welcher in einen weniger gekrümmten Teilzylinder bzw. in wenigstens eine ebene Fläche übergeht, aufweisen, sowie für die Ausführungsform, welche in einer ersten Schnittebene eine im Wesentlichen V-förmige Gestalt hat.

Die Lagerung, d. h. Stapelbarkeit, wird in besonderem Maße bei jenen Ausführungsformen verbessert, welche in ihrer dreidimensionalen Form mit wenigstens einer, zumindest annähend ebenen Fläche versehen sind. Gleiches gilt, wenn die dreidimensionale Form in Gestalt eines Teilzylinders, welcher in einen wesentlich weniger gekrümmten Teilzylinder übergeht, vorliegt.

Für die Abdeckfunktion des dentalen Einlegemittels sind in besonderem Maße jene Ausführungsformen vorteilhaft, welche in ihrer dreidimensionalen Form mit wenigstens einer, zumindest annähend ebenen Fläche versehen sind. Gleiches gilt, wenn die dreidimensionale Form in Gestalt eines Teilzylinders, welcher in einen wesentlich weniger gekrümmten Teilzylinder übergeht, vorliegt. Als besonders vorteilhaft ist in diesem Zusammenhang jene Ausführungsform anzusehen, welche wenigstens eine Fläche aufweist, welche der Kontur anatomischer Gegebenheiten in der Mundhöhle angepasst ist.

Ferner kann das erfindungsgemäße dentale Einlegemittel vorteilhaft so ausgebildet werden, dass ein Verkleben des dentalen Einlegemittels mit anatomischen Strukturen der Mundhöhle weitestgehend verhindert werden kann.

Ferner betrifft die Erfindung ein Endlosband, von welchem das erfindungsgemäße dentale Einlegemittel in einer gewünschten Länge abgelängt werden kann.

Die Erfindung wird nun anhand von Ausführungsbeispielen näher erläutert, wobei Bezug auf die beigefügten Zeichnungen genommen wird. Es zeigen
Fig. 1 eine perspektivische Ansicht einer Ausführungsform des erfindungsgemäßen dentalen Einlegemittels.
Fig. 2A und 2B eine weitere Ausführungsform des erfindungsgemäßen dentalen Einlegemittels, wobei Fig. 2A eine Schnittansicht gemäß der Linie II-II von Fig. 2B ist, und Fig. 2B eine Schnittansicht gemäß der Linie I-I von Fig. 2A ist.

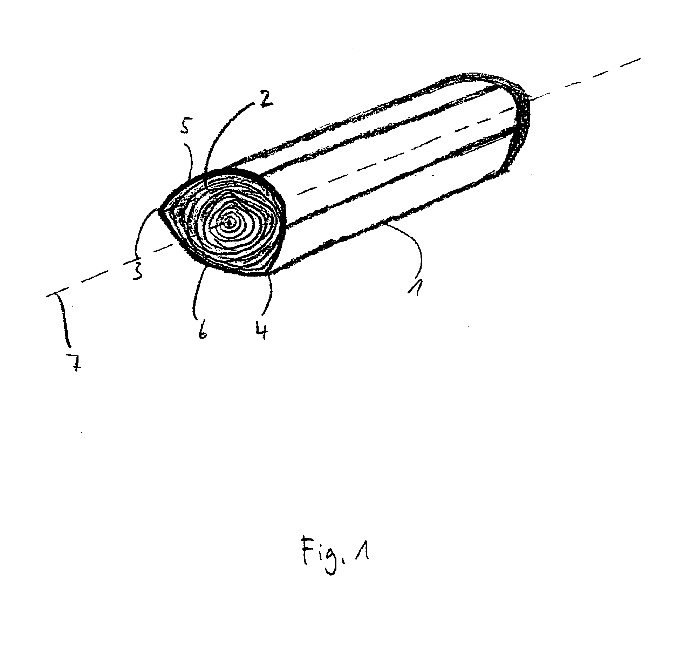

Fig. 1 zeigt eine aus Baumwollewatte gefertigte, erste Ausführungsform des dentalen Einlegemittels 1 gemäß der Erfindung. Das dentale Einlegemittel 1 liegt in Form eines Längsprofilstücks mit der die Längsrichtung definierenden Längsachse 7 vor. Die senkrecht zur Längsachse geschnittene Querschnittsfläche 2 des Längsprofilstücks weist einen geometrischen Umfang auf, welcher sich aus einer ersten Kreislinie 5 und einer zweiten Kreislinie 6 zusammensetzt. Hierbei weist die zweite Kreislinie 6 einen wesentlich größeren Krümmungsradius auf als die erste Kreislinie 5.

Die beiden Kreislinien 5, 6 gehen jeweils in einer Knickstelle 3, 4 ineinander über. In der dreidimensionalen Form setzt sich das dentale Einlegemittel, entsprechend den ersten und zweiten Kreislinien 5, 6 aus zwei parallel zu deren jeweiligen Zylinderachse geschnittenen Teilzylindern zusammen. Die als Knickstellen 3, 4 ausgebildeten Verbindungen der ersten und zweiten Kreislinie 5, 6 entsprechen in der dreidimensionalen Form jeweiligen Kanten der äußeren Oberfläche des dentalen Einlegemittels.

Das in Fig. 1 gezeigte dentale Einlegemittel kann in einfacher Weise durch Rollen und Verkleben einer Lage von Baumwollwatte, sowie anschließendes Verpressen der Watterolle zum Zwecke der Formgebung des dentalen Einlegemittels in der angegebenen Weise, hergestellt werden. Ebenso kann das dentale Einlegemittel in Form eines Endlosbands, welches über geeignete Formsysteme, wie etwa dreieckige Trichter und weitere Formhilfen, in erfindungsgemäßer Weise verformt wird, und anschließendes Umhüllen mit einem Vlies zum Zwecke der Formstabilität, hergestellt werden.

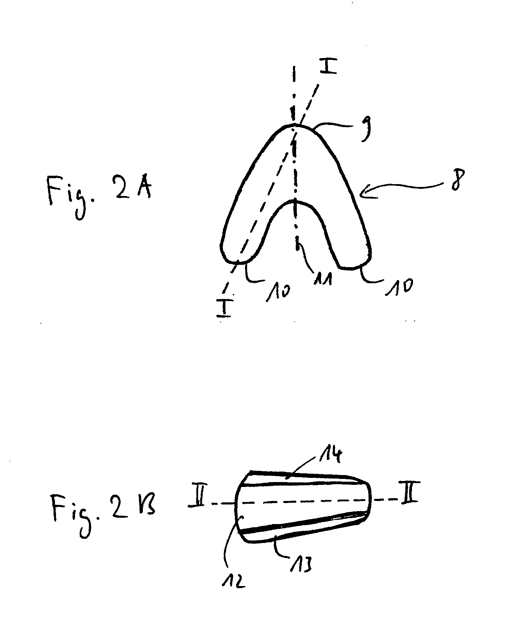

Fig. 2A und 2B zeigen eine weitere Ausführungsform des erfindungsgemäßen dentalen Einlegemittels, wobei Fig. 2A einer Schnittansicht gemäß der Linie II-II von Fig. 2B entspricht, während Fig. 2B einer Schnittansicht gemäß der Linie I-I von Fig. 2A entspricht. Wie Fig. 2A zu entnehmen ist, weist das dentale Einlegemittel 8 in der Draufsicht, welcher einer ersten Schnittebene (II-II) entspricht, im Wesentlichen eine V-förmige Gestalt auf. In einer zur ersten Schnittebene senkrechten Schnittebene (I-I) ist das dentale Einlegemittel keilförmig geformt. Wie Fig. 2A zu entnehmen ist, ergibt sich die im Wesentlichen V-förmige Gestalt des dentalen Einlegemittels 8 aus einem Spitzenabschnitt 9, sowie zwei Schenkelabschnitten 10. Bezüglich einer Mittenebene 11 ist das dentale Einlegemittel symmetrisch ausgebildet. Wie sich aus Fig. 2B ergibt, ist das dentale Einlegemittel aus einem mehrlagigen Verbundmaterial geformt, wobei das Verbundmaterial aus einer keilförmigen Kernlage 12 aus Silikon und die Kernlage 12 umgebenden, saugfähigen Decklagen 13 bzw. 14 aus Viskose und Baumwolle zusammengesetzt ist. Die Decklagen 13 bzw. 14 sind auf die Kernlage 12 mittels eines geeigneten Klebstoffs aufgeklebt. Die in Fig. 2A und 2B gezeigte Ausführungsform des dentalen Einlegemittels ist besonders geeignet, unter der Zunge platziert zu werden, um die Zunge nach oben zu verschieben und Platz für den behandelnden Zahnarzt zu schaffen.

## Patentansprüche

1. Dentales Einlegemittel zur Anwendung in der Mundhöhle, **dadurch gekennzeichnet, dass** es eine Querschnittsfläche aufweist, die
a) einen Umfang in Gestalt eines ersten Teilkreises mit einem ersten Krümmungsradius aufweist, dessen Endpunkte durch wenigstens eine gekrümmte Linie verbunden sind, oder
b) einen Umfang in Gestalt eines ersten Teilkreises aufweist, dessen Endpunkte durch wenigstens eine wenigstens annähernd gerade Linie verbunden sind, oder
c) einen Umfang in Gestalt eines geschlossenen Polygonzugs aufweist,
und/oder
d) eine im Wesentlichen'V-förmige Gestalt aufweist und für diesen Fall (d) eine in Längsrichtung sich verjüngende dreidimensionale Form aufweist.

2. Dentales Einlegemittel nach Anspruch 1, **dadurch gekennzeichnet, dass** es eine Querschnittsfläche mit einem Umfang in Gestalt eines ersten Teilkreises mit einem ersten Krümmungsradius aufweist, dessen Endpunkte durch wenigstens eine gekrümmte Linie verbunden sind.

3. Dentales Einlegemittel nach Anspruch 2, **dadurch gekennzeichnet, dass** die die Endpunkte des ersten Teilkreises verbindende gekrümmte Linie in Gestalt eines zweiten Teilkreises vorliegt, wobei der Krümmungsradius des ersten Teilkreises kleiner ist, als der Krümmungsradius des zweiten Teilkreises.

4. Dentales Einlegemittel nach Anspruch 1, **dadurch gekennzeichnet, dass** es eine Querschnittsfläche mit einem Umfang in Gestalt eines ersten Teilkreises aufweist, dessen Endpunkte durch wenigstens eine wenigstens annähernd gerade Linie verbunden sind.

5. Dentales Einlegemittel nach einem der vorhergehenden Ansprüche 2 bis 4, **dadurch gekennzeichnet, dass** die die Endpunkte des ersten Teilkreises verbindende Linie in einer wenigstens annähernd nicht stetig differenzierbaren Weise in den ersten Teilkreis übergeht.

6. Dentales Einlegemittel nach Anspruch 1, **dadurch gekennzeichnet, dass** es eine Querschnittsfläche mit einem Umfang in Gestalt eines geschlossenen Polygonzugs aufweist.

7. Dentales Einlegemittel nach Anspruch 6, **dadurch gekennzeichnet, dass** der Umfang der Querschnittsfläche eine Bienenwabenstruktur aufweist.

8. Dentales Einlegemittel nach Anspruch 6, **dadurch gekennzeichnet, dass** der Umfang der Querschnittsfläche eine Rechteckform aufweist.

9. Dentales Einlegemittel nach Anspruch 6, **dadurch gekennzeichnet, dass** der Umfang der Querschnittsfläche eine Dreiecksform aufweist.

10. Dentales Einlegemittel nach einem der vorhergehenden Ansprüche, **dadurch gekennzeichnet, dass** es eine in Längsrichtung sich verjüngende dreidimensionale Form aufweist.

11. Dentales Einlegemittel nach einem der vorhergehenden Ansprüche, **dadurch gekennzeichnet, dass** es aus Watte gefertigt ist.

12. Dentales Einlegemittel nach einem der vorhergehenden Ansprüche, **dadurch gekennzeichnet, dass** es in Form eines Längsprofilstücks vorliegt.

13. Dentales Einlegemittel nach einem der vorhergehenden Ansprüche, **dadurch gekennzeichnet, dass** die Querschnittsfläche senkrecht zur Längsrichtung geschnitten ist.

14. Dentales Einlegemittel nach einem der vorhergehenden Ansprüche, **dadurch gekennzeichnet, dass** es keine Querschnittsfläche mit einem kreisförmigen Umfang aufweist.

15. Dentales Einlegemittel nach einem der vorhergehenden Ansprüche, **dadurch gekennzeichnet, dass** es eine gerade oder gekrümmte dreidimensionale Form aufweist.

16. Dentales Einlegemittel nach Anspruch 10, **dadurch gekennzeichnet, dass** es in einer ersten Schnittebene eine im Wesentlichen V-förmige Gestalt aufweist.

17. Dentales Einlegemittel nach Anspruch 16, **dadurch gekennzeichnet, dass** es in einer zur ersten Schnittebene senkrechten zweiten Schnittebene im Wesentlichen keilförmig ausgebildet ist.

18. Dentales Einlegemittel nach Anspruch 16 oder 17, **dadurch gekennzeichnet, dass** es aus einer Materialkomponente, vorzugsweise aus einem geschäumten Material, oder aus mehreren Materialkomponenten geformt ist, vorzugsweise künstlicher Faser oder natürlicher Faser, gegebenenfalls kombiniert mit hoch saugfähigem Stoff.

19. Dentales Einlegemittel nach Anspruch 18, **dadurch gekennzeichnet, dass** es aus einem mehrlagigen Verbundmaterial geformt ist.

20. Dentales Einlegemittel nach Anspruch 19, **dadurch gekennzeichnet, dass** das Verbundmaterial aus einer Kernlage und die Kernlage umgebenden Decklagen zusammengesetzt ist.

21. Dentales Einlegemittel nach Anspruch 19 oder 20, **dadurch gekennzeichnet, dass** das Kernmaterial aus wenigstens einem Material aus der Gruppe, bestehend aus Silikon, Naturfasern, Synthetikfasern und Kunststoffen, insbesondere Polyethylen, Polyamid oder Polypropylen und gegebenenfalls üblichen Zusatzstoffen, gewählt ist, wobei das Kernmaterial saugfähig oder hoch saugfähig sein kann, während die Decklagen unabhängig voneinander aus wenigstens einem Material aus der Gruppe, bestehend aus gegebenenfalls saugfähigen natürlichen Fasern und gegebenenfalls saugfähigen synthetischen Fasern und gegebenenfalls üblichen Zusatzstoffen, gewählt sind, wobei die Decklagen feuchtigkeitsdurchlässig sind und den Feuchtigkeitstransport von außen in das Innere des Einlegemittels nicht behindern.

22. Dentales Einlegemittel nach einem der vorhergehenden Ansprüche, **dadurch gekennzeichnet, dass** es wenigstens eine Fläche aufweist, welche anatomischen Gegebenheiten in der Mundhöhle, insbesondere der Kontur von einem oder mehreren Zähnen, der Kontur der Zunge, oder der Kontur der Innenwand der Mundhöhle wie dem oberen oder unteren Gaumenbereich, angepasst ist.

23. Endlosband, welches eine Vielzahl von dentalen Einlegemitteln nach einem der vorhergehenden Ansprüche umfasst.

## Claims

1. Dental insertion element for use in the oral cavity, **characterised in that** it has a cross-sectional area
a) having a periphery in the shape of a first part-circle, having a first radius of curvature, the end-points of which are joined by at least one curved line, or
b) having a periphery in the shape of a first part-circle the end-points of which are joined by at least one at least approximately straight line,
or
c) having a periphery in the shape of a closed polygonal figure,
and/or
d) which is substantially V-shaped and in this case (d) it has a three-dimensional shape that becomes narrower in the longitudinal direction.

2. Dental insertion element according to claim 1, **characterised in that** it has a cross-sectional area having a periphery in the shape of a first part-circle, having a first radius of curvature, the end-points of which are joined by at least one curved line.

3. Dental insertion element according to claim 2, **characterised in that** the curved line joining the end-points of the first part-circle is in the shape of a second part-circle, the radius of curvature of the first part-circle being smaller than the radius of curvature of the second part-circle.

4. Dental insertion element according to claim 1, **characterised in that** it has a cross-sectional area having a periphery in the shape of a first part-circle the end-points of which are joined by at least one at least approximately straight line.

5. Dental insertion element according to one of the preceding claims 2 to 4, **characterised in that** the line joining the end-points of the first part-circle passes into the first part-circle in an at least approximately not continuously differentiable manner.

6. Dental insertion element according to claim 1, **characterised in that** it has a cross-sectional area having a periphery in the shape of a closed polygonal figure.

7. Dental insertion element according to claim 6, **characterised in that** the periphery of the cross-sectional area has a honeycomb structure.

8. Dental insertion element according to claim 6, **characterised in that** the periphery of the cross-sectional area has a rectangular shape.

9. Dental insertion element according to claim 6, **characterised in that** the periphery of the cross-sectional area has a triangular shape.

10. Dental insertion element according to one of the preceding claims, **characterised in that** it has a three-dimensional shape that becomes narrower in the longitudinal direction.

11. Dental insertion element according to one of the preceding claims, **characterised in that** it is produced from wadding.

12. Dental insertion element according to one of the preceding claims, **characterised in that** it is in the shape of a longitudinal profile part.

13. Dental insertion element according to one of the preceding claims, **characterised in that** the cross-sectional area is the cross-sectional area cut perpendicular to the longitudinal direction.

14. Dental insertion element according to one of the preceding claims, **characterised in that** it has no cross-sectional area having a circular periphery.

15. Dental insertion element according to one of the preceding claims, **characterised in that** it has a straight or curved three-dimensional shape.

16. Dental insertion element according to claim 10, **characterised in that**, in a first sectional plane, it is substantially V-shaped.

17. Dental insertion element according to claim 16, **characterised in that**, in a second sectional plane perpendicular to the first sectional plane, it is substantially wedge-shaped.

18. Dental insertion element according to claim 16 or 17, **characterised in that** it is formed from one material component, preferably a foamed material, or from a plurality of material components, preferably synthetic fibre or natural fibre, optionally combined with highly absorbent material.

19. Dental insertion element according to claim 18, **characterised in that** it is formed from a multi-layer composite material.

20. Dental insertion element according to claim 19, **characterised in that** the composite material is composed of a core layer and covering layers surrounding the core layer.

21. Dental insertion element according to claim 19 or 20, **characterised in that** the core material is selected from at least one material from the group consisting of silicone, natural fibres, synthetic fibres and plastics, especially polyethylene, polyamide or polypropylene, and, optionally, customary additives, it being possible for the core material to be absorbent or highly absorbent, whereas the covering layers independently of one another are selected from at least one material from the group consisting of optionally absorbent natural fibres and optionally absorbent synthetic fibres and, optionally, customary additives, the covering layers being permeable to moisture and not impeding the transport of moisture from the outside to the interior of the insertion element.

22. Dental insertion element according to one of the preceding claims, **characterised in that** it has at least one surface which is matched to anatomical features in the oral cavity, especially to the contour of one or more teeth, to the contour of the tongue, or to the contour of the inner wall of the oral cavity such as the upper or lower palate region.

23. Endless strand comprising a multiplicity of dental insertion elements according to one of the preceding claims.

## Revendications

1. Inlay dentaire destiné à être utilisé dans la cavité buccale, **caractérisé en ce qu'**il présente une face de section transversale, qui
a) présente un contour sous la forme d'un premier cercle partiel doté d'un premier rayon de courbure, dont les points terminaux sont reliés par au moins une ligne courbée,
ou
b) présente un contour sous la forme d'un premier cercle partiel, dont les points terminaux sont reliés par au moins une ligne au moins de manière approchante rectiligne,
ou
c) présente un contour sous la forme d'un tracé polygonal fermé,
et/ou
d) présente une forme essentiellement en V et, dans le cas présent (d), une forme tridimensionnelle se rétrécissant dans la direction longitudinale.

2. Inlay dentaire selon la revendication 1, **caractérisé en ce qu'**il présente une face de section transversale ayant un contour sous la forme d'un premier cercle partiel présentant un premier rayon de courbure, dont les points terminaux sont reliés par au moins une ligne courbée.

3. Inlay dentaire selon la revendication 2, **caractérisé en ce que** la ligne courbée reliant les points terminaux du premier cercle partiel est présente sous la forme d'un deuxième cercle partiel, sachant que le rayon de courbure du premier cercle partiel est inférieur au rayon de courbure de deuxième cercle partiel.

4. Inlay dentaire selon la revendication 1, **caractérisé en ce qu'**il présente une face de section transversale dotée d'un contour sous la forme d'un premier cercle partiel, dont les points terminaux sont reliés par au moins une ligne au moins de manière approchante rectiligne.

5. Inlay dentaire selon l'une quelconque des revendications précédents 2 à 4, **caractérisé en ce que** la ligne reliant les points terminaux du premier cercle partiel se prolonge dans le premier cercle partiel d'une manière ne pouvant pas être différencié systématiquement au moins de manière approchante.

6. Inlay dentaire selon la revendication 1, **caractérisé en ce qu'**il présente une face de section transversale dotée d'un contour ayant la forme d'un tracé polygonal fermé.

7. Inlay dentaire selon la revendication 6, **caractérisé en ce que** le contour de la face de section transversale présente une structure alvéolaire.

8. Inlay dentaire selon la revendication 6, **caractérisé en ce que** le contour de la face de section transversale présente une forme rectangulaire.

9. Inlay dentaire selon la revendication 6, **caractérisé en ce que** le contour de la face de section transversale présente une forme triangulaire.

10. Inlay dentaire selon l'une quelconque des revendications précédentes, **caractérisé en ce qu'**il présente une forme tridimensionnelle se rétrécissant dans la direction longitudinale.

11. Inlay dentaire selon l'une quelconque des revendications précédentes, **caractérisé en ce qu'**il est fabriqué à partir de ouate.

12. Inlay dentaire selon l'une quelconque des revendications précédentes, **caractérisé en ce qu'**il se présente sous la forme d'une pièce profilée longitudinale.

13. Inlay dentaire selon l'une quelconque des revendications précédentes, **caractérisé en ce que** la face de section transversale est coupée de manière perpendiculaire par rapport à la direction longitudinale.

14. Inlay dentaire selon l'une quelconque des revendications précédentes, **caractérisé en ce qu'**il ne présente aucune face de section transversale dotée d'un contour circulaire.

15. Inlay dentaire selon l'une quelconque des revendications précédentes, **caractérisé en ce qu'**il présente une forme tridimensionnelle rectiligne ou courbée.

16. Inlay dentaire selon la revendication 10, **caractérisé en ce qu'**il présente, dans un premier plan de coupe, une forme essentiellement en V.

17. Inlay dentaire selon la revendication 16, **caractérisé en ce qu'**il est réalisé, dans un deuxième plan de coupe perpendiculaire par rapport au premier plan de coupe, essentiellement de manière à présenter une forme conique.

18. Inlay dentaire selon la revendication 16 ou 17, **caractérisé en ce qu'**il est formé à partir d'un composant de matériau, de préférence à partir d'un matériau expansé, ou à partir de plusieurs composants de matériau, de préférence à fibres synthétiques ou à fibres naturelles, éventuellement en combinaison avec une matière hautement absorbante.

19. Inlay dentaire selon la revendication 18, **caractérisé en ce qu'**il est formé à partir d'un matériau composant multicouche.

20. Inlay dentaire selon la revendication 19, **caractérisé en ce que** le matériau composite est composé d'une couche centrale et de couches de recouvrement entourant la couche centrale.

21. Inlay dentaire selon la revendication 19 ou 20, **caractérisé en ce que** le matériau central est choisi parmi au moins un matériau issu du groupe comprenant le silicone, les fibres naturelles, les fibres synthétiques et les matières plastiques, en particulier le polyéthylène, le polyamide ou le polypropylène, et éventuellement d'autres additifs supplémentaires, sachant que le matériau central peut être absorbant ou hautement absorbant, tandis que les couches de recouvrement sont choisies, indépendamment les unes des autres, parmi au moins un matériau issu du groupe comprenant éventuellement des fibres naturelles absorbantes et éventuellement des fibres synthétiques absorbantes et éventuellement d'autres additifs, sachant que les couches de recouvrement sont perméables à l'humidité et n'empêchent pas l'infiltration de l'humidité en provenance de l'extérieur à l'intérieur de l'inlay.

22. Inlay dentaire selon l'une quelconque des revendications précédentes, **caractérisé en ce qu'**il présente au moins une face, qui est adaptée aux données anatomiques de la cavité buccale, en particulier au contour d'une ou de plusieurs dents, au contour de la langue, ou au contour de la paroi intérieure de la cavité buccale telle que le palais supérieur ou inférieur.

23. Bande sans fin, qui comprend une pluralité d'inlays dentaires selon l'une quelconque des revendications précédentes.
